Europäisches Patentamt

European Patent Office

Office européen des brevets

(11) Publication number: **0 511 759 A1**

## EUROPEAN PATENT APPLICATION

(21) Application number: 92303460.7

(22) Date of filing: 16.04.92

(51) Int. Cl.5: **C12Q 1/18**, C12Q 1/02

(30) Priority: **29.04.91 US 693982**

(43) Date of publication of application:
**04.11.92 Bulletin 92/45**

(84) Designated Contracting States:
**BE DE FR GB NL**

(71) Applicant: **THE STANDARD OIL COMPANY**
**200 Public Square, 7A**
**Cleveland, Ohio 44114-2375(US)**

(72) Inventor: **Horowitz, Amikam**
**3666 Stoer Avenue**
**Shaker Heights, Ohio 44122(US)**

(74) Representative: **Ryan, Edward Terrence et al**
**BP INTERNATIONAL LIMITED Patents &**
**Agreements Division Chertsey Road**
**Sunbury-on-Thames Middlesex, TW16**
**7LN(GB)**

(54) Biotoxicity test for chemicals.

(57) The toxic effect of a chemical is determined by culturing a sample of protozoa in the presence of a chemical and determining the amount of remaining feed after an incubation period.

EP 0 511 759 A1

FIELD OF THE INVENTION

The present invention relates to a method of determining the toxic potential of chemicals by a bioassay. Further the invention relates to a sensitive and rapid bioassay for determining the toxic effect of chemicals.

BACKGROUND OF THE INVENTION

There exists a need for determining the toxicity of chemicals. Measurement of the potential toxic effects of chemicals is critical for the protection of the people that come in contact with the chemicals such as production workers and consumers. Further, toxicity testing is important in the protection of the environment. Toxicity testing is and will be required by federal and state regulatory agencies because of increasing environmental and medical concerns. Toxicity testing required by regulatory agencies, however, is often expensive.

Previously known toxicity tests have employed vertebrate and invertebrate organisms. Many of the tests involve complex, multi-cellular organisms, such as the Daphnids and Ceriodaphnids. Generally, the methods rely on the direct counting or observations of a test organism and the effect of a toxicant on the test organism. The tests directly monitor inhibition in multiplication, decrease in population or death of the test organisms on exposure to the toxicant.

Rogerson, A., W.Y. Shiu, G.L. Huang, D. Mackay, and J. Berger. 1983. Determination And Interpretation Of Hydrocarbon Toxicity To Ciliate Protozoa. Aquatic Toxicology, 3: 215-228 discloses acute toxicity threshold of hydrocarbons on two species of ciliate protozoa. This article discloses the sensitive nature of protozoa for ecotoxicity measurements.

Bringmann, G. and R. Kuhn. 1980A, Comparison Of The Toxicity Thresholds Of Water Pollutants To Bacteria, Algae, And Protozoa In The Cell Multiplication Inhibition Test, Water Res. 14: 231-241. This article compares bioassays for determining the toxicity thresholds of water pollutants using Pseudomonas putida, a bacterium; Scendesmus quadricauda, a green alga; and Entosiphon sulcatum a protozoan. The inhibition of cell multiplication is determined by turbidimetry for the bacteria and the algae at 436 nm and 578 nm, respectively, and by using a cell counter to measure the number of protozoa.

Bringmann, G. and R. Kuhn. 1980B, Determination Of The Harmful Biological Effect Of Water Pollutants On Protozoa. II, Bacteriovorous Ciliates. Z. Wasser. Abwasser Forsch. 13: 26-31 describes a measurement of the toxicity threshold levels of various water pollutants by feeding a pure strain of Escherichia coli bacteria to cultures of bacteriovorous ciliate protozoan, Uronema parduczi and then quantitatively measuring the protozoan cell population and multiplication of the protozoa using an electronic cell counter. This method measures the number of directly affected organisms and not the feed.

Slabbert, J.L. and W.S.G. Morgan. 1982, A Bioassay Technique Using Tetrahymena Pyiformis For The Rapid Assessment Of Toxicants In Water, Water Res. 16: 517-523 discloses a bioassay based on changes in oxygen uptake rate of the protozoa Tetrahymena pyriformis following exposure to a variety of toxicants.

Dutka, B.J., N. Nyholm and J. Petersen, 1983, Comparison Of Several Microbiological Toxicity Screening Tests, Water Res. 17: 1363-1368 compares Beckman's Microtox microbiological toxicity screening test, which utilizes specialized strains of luminescent bacteria of Photobacterium phosphoreum with the Spirillum volutans test, which uses a large aquatic bacterium. These test methods measure the decrease in bacteria population or loss in bacterial motility.

Alsop, G.M., G.T. Waggy and R.A. Conway, 1980, Bacterial Growth Inhibition Test. J. Water Pollut. Control Fed. 52: 2452-2456 and Munson, R.J. 1970, Turbidostats, In Methods In Microbiology, edited by J.R. Norris and D.W. Ribbons, Academic Press, Vol. 2: 350-353 describes a growth inhibition test of using turbidity, optical density (absorbance) or light scattering as indicators of populations of bacteria.

U.S.P.N. 4,604,351 teaches a method for determining bacterial sensitivity to chemical agents.

U.S.P.N. 3,981,777 discloses a method and apparatus for testing materials for levels of agents inhibitory or toxic to microorganisms.

There is a need to find more efficient, effective, sensitive and inexpensive bioassays which measures the potential toxic effect of chemicals. The present invention has discovered a simple, rapid and sensitive method to determine the effect of a chemical on the physiological behavior of a protozoa test organism. The present inventive method monitors the toxic effect of a chemical on an organism by measuring the disappearance of its feed rather then monitoring the organism.

The present invention has discovered that protozoa, relatively simple microorganisms, are as sensitive to toxicants as are the more complex, multicellular organisms, such as the Daphnids. The present invention is simple, inexpensive and easy to perform. The present method does not require sophisticated instrumentation and can readily be automated.

It is an object of the invention to provide a bioassay to determine the toxicity of chemicals. It is a further object of the invention to provide a rapid, simple and sensitive method to determine the biotoxicity of chemicals employing microorganisms. It is an additional object of the invention to provide for the use of simple microorganisms of protozoa and not more complex multicellular organisms, with similar sensitivity to toxicants in the bioassay.

These and other objects, together with the advantages over known methods, shall become apparent from the specification which follows and are accomplished by the invention as herein described and claimed.

## BRIEF DESCRIPTION OF THE FIGURES

Figure 1 - Correlation between light absorbance at 540 nm and the number of bacterial cells.
Figure 2 - Impact of live protozoa on bacterial growth.
Figure 3 - Effect of protozoa on dead bacterial cells.
Figure 4 - Effect of Igepal surfactant on live bacterial-protozoan culture.
Figure 5 - Effect of biological wastewater treatment system on live bacterial-protozoan culture.

## SUMMARY OF THE INVENTION

A process has been discovered whereby the biotoxic effect of chemicals can be determined on microorganisms by a rapid and sensitive bioassay.

A process has been discovered for determining the biotoxic effect of a chemical on organisms comprising:

a) obtaining protozoa at concentrations insufficient to scatter greater than about 2% of incident light at about 540 nm, i.e., absorbance of about <0.02;

b) adding an organic feed to the protozoa to form a growing protozoa culture, wherein the feed comprises;

    i) at least one substance at a concentration sufficient to scatter light of wavelengths greater than 400 nm, or

    ii) at least one substance at an initial concentration insufficient to scatter light of wavelengths greater than 400 nm, but which can increase in concentration sufficiently to scatter light of wavelengths greater than 400 nm;

c) dividing the protozoa culture and the feed into a test sample and a control sample;

d) adding to the test sample a chemical having unknown toxic effect at a concentration sufficient to alter the feeding behavior of the protozoa;

e) culturing the test sample containing the added chemical and the control sample without the added chemical under similar conditions until more than about 70% of the light-scattering feed has been consumed in the control sample;

f) determining the concentrations of the light-scattering feed remaining in the test and control samples, wherein the determination can be carried out by either a visual examination or by measurement of absorbance at a selected wavelength; and

g) relating the relative concentrations of the remaining feed to the toxicity of the chemical agent.

The process of the instant invention is useful in determining the toxic effect of chemicals on organisms. The invention can be used for screening pure chemicals and mixtures of chemicals by any industrial plant or laboratory facility that carries out toxicity testing on chemicals, products or wastes.

## DESCRIPTION OF THE INVENTION

The instant invention describes a rapid and sensitive biotoxicity test for chemicals. The instant invention is an assay to determine the biotoxicity of a chemical on protozoa. The bioassay employs at least two separate samples containing a protozoa population and at least one organic feed cultured under controlled conditions. The samples are cultured in an aqueous medium including growth factors. The test sample includes a chemical agent having potential toxic effect on the protozoa in the aqueous medium. The other sample (control sample) does not include the chemical agent. The test and control samples are cultured and the amount of feed present in each medium is determined by visual examination of turbidity or by spectral measurement, and then the respective concentrations of feed are compared, resulting in a determination of biotoxicity of the tested chemical.

Many protozoa such as Anthophysa vegetans (also known as Anthophysis vegetans), feed by ingesting small organic particles. Protozoa can also feed by preying on smaller organisms, such as bacteria, which can be either live or dead. Bacteria and protozoa represent consecutive steps in the food chain. Bacteria grow and reproduce more rapidly than protozoa under similar conditions and provide a food source for the protozoa. Bacteria are significantly less sensitive to toxic chemicals than the protozoa; and as a result, on exposure to a toxicant, protozoan feeding behavior will be affected prior to any impact on the bacteria.

Generally, under normal growth conditions, protozoa consume organic feed and if the amount of feed is limited, then after the available feed is consumed the growth of the protozoa will cease. When the protozoa encounter adverse conditions, such as toxicants, prior to consuming all available feed, the protozoa will cease feeding and no change in the amount of remaining feed will be observed. The difference between favorable conditions, that is, the chemical does not have a toxic effect on the protozoa and unfavorable or adverse growth conditions can be assessed by the instant invention by measuring the amount of feed remaining in the medium after a specific time. The toxicants affect the feeding behavior of the protozoa. There is greater inhibition of protozoan feeding as the toxicity of the chemical increases. The impact of the toxicants on the feeding behavior of the protozoa is measured by the amount of the feed consumed by the protozoa. When the protozoa are affected by the toxicant, they do not consume the feed and the turbidity does not change. When the protozoa are not affected by the added toxicant, the protozoa consume the feed and the turbidity of the medium is reduced. The concentration of the organic feed and not the protozoa is measured as indicated by the solution turbidity.

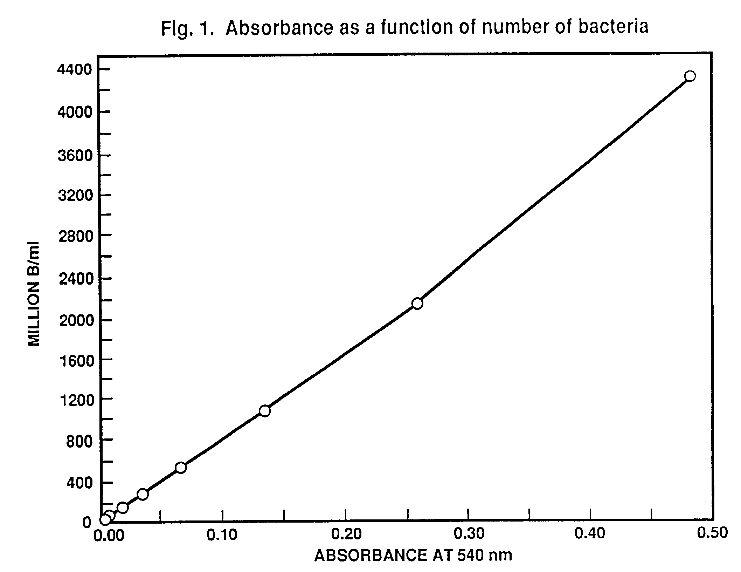

In accordance with the invention, the activity of the protozoa is measured as expressed in their feeding habits. The number of protozoa, alive or dead, is not measured; rather the amount of their feed, the bacteria or organic particles, is determined based on the turbidity of the culture medium. The measurement of the bacterial population or organic feed concentration is a rapid and sensitive test since it determines the effect of a chemical on the physiological behavior of the test organism, the protozoa, rather than recording the death or inhibition in multiplication of the test organism. The method of the present invention differs from other bioassays that use microorganisms to assess the biotoxicity of a chemical in that the toxic effect on the test microorganism is not measured directly by determining the inhibition in multiplication, decrease in population or death of the test microorganism. In the instant invention, the protozoa's ability to feed, a physiological trait, is monitored. The impact on the protozoa of the added chemical is determined by measuring the concentration of feed remaining after a given period of incubation in a sample containing added chemical of unknown toxicity. The concentration of the feed is measured by the turbidity or lack of turbidity in liquid media containing the protozoa and feed. The turbidity indicates that the chemical was toxic to or inhibited the feeding behavior of the protozoa whereas clearness or reduced turbidity indicates that the feeding behavior of the protozoa was unaffected or minimally affected by the added chemical. Figure 1 shows a linear correlation is observed between absorbance at 540 nm and bacterial concentration over the range of about $1.8 \times 10^7$ bacteria per ml and about $4.3 \times 10^9$ bacteria per ml. A linear relation is shown between the number of bacteria and turbidity as measured by light absorbance.

When a sufficient concentration of feed such as bacteria or organic particles are present in an aqueous solution, the culture medium becomes cloudy or turbid in the visible range. During normal feeding, the protozoa consume the feed of the bacterial cells or organic particles resulting in decreased turbidity. The turbidity can be seen visually or can be measured using a spectrophotometer in the visible light range of about 350 nm to about 650 nm, preferably at a wavelength of about 540 nm.

The protozoa test organism needs to be sensitive and should be convenient to use. The protozoa useful for this bioassay include but are not limited to protozoa in the genera Anthophysa, Labyrinthula, Paramediium and the like. In particular, the protozoa include but are not limited to Anthophysa vegetans, Labyrinthula macrocystis, Paramecium aurelia, Tetrahymena puriformis and the like. The protozoa preferably employed in the method of the instant invention are Anthophysa vegetans. The protozoa can be used as a pure culture or a mixed protozoa culture. Generally, the protozoa are obtained from soils, water bodies and the like. When a pure protozoan population is employed, a tailored prey population may be used to enhance somewhat the growth rate of the protozoa.

The feed for the protozoa include but are not limited to non-living organic particles, living particles and the like. Preferably the feed is bacterial cells that are living or dead. The non-living organic particles are either from a biological or a non-biological origin. Typical non-living organic particles include but are not limited to dead bacteria which may be killed by any method such as age, malfunction, heat, radiation and the like; cellulose granules; starch granules; oil droplets; and the like. Typical living organic particles include but are not limited to bacteria and the like. Bacteria include, but are not limited to Acinetobacter, Corynebacterium, Enterobacter, Psuedomonas and the like. The bacteria can be employed as a pure culture or mixed population of bacteria. Generally, the bacteria are obtained from soils, water bodies and the like. When bacteria are used as feed for the protozoa the bacteria need to be prey for the protozoa employed in the bioassay.

The non-living and/or living organic particles are added to the sample at a concentration sufficient to increase the turbidity of the medium to at least about 0.3 at 540 nm in about a 1.0 cm pathlength tube. When unaffected by the toxicants, the protozoa consume the organic particles during their growth phase and sample turbidity is reduced. When the protozoa are affected by the toxicant, their feeding behavior is inhibited and they do not consume the organic particle feed and the turbidity of the protozoa-organic particle-toxicant sample does not change.

The living organic particles, such as bacteria, can be added at high or low concentrations. The living organic particles such as bacteria can be added as feed concentrations at greater than about $2.5 \times 10^9$ per ml, indicated by a turbidity of the bacterial culture of at least about 0.3 at 540 nm in a 1.0 cm pathlength tube, in this instance, the bacteria are no longer growing due to a limited amount of nutrient in the culture medium. Generally the live bacteria can be added as feed concentrations in the range of about $1 \times 10^6$ per ml to about $1 \times 10^8$ per ml, and preferably less than about $7 \times 10^7$ per ml which is indicated by a turbidity of less than 0.1 at 540 nm in a 1.0 cm pathlength tube, and the bacteria are growing by consuming the added nutrients. Theoretically, the live bacteria can be added as feed concentrations as low as one viable bacterium in the test tube. When live bacteria in this concentration range are used as feed, the bacteria need to reproduce and grow more rapidly than the protozoa under similar conditions and provide a food source for the protozoa. As the bacteria grow in number, they scatter more light and the culture medium becomes turbid.

The bioassay of this invention has value in determining the toxic effect of chemical agents including but not limited to pharmaceuticals, agrochemical products, chemicals, pesticides, industrial effluents, pollutants, waste, wastewater and the like. Chemical agents which have specific bactericidal action, such as antibiotics, need to employ non-living feed in the test sample.

The chemical agent to be tested is in liquid form or put into an aqueous suspension prior to being subjected to the bioassay.

The toxic effect of the chemical agent is evaluated in an aqueous medium containing inorganic buffers such as Bushnell-Haas broth; nutrients such as carbon sources of glucose, yeast extract and the like; protozoa; organic feed and the like.

## SPECIFIC EMBODIMENTS

This invention is further illustrated by the following examples, although it is understood that the examples are intended merely for purposes of illustration and are not intended to limit the scope of the invention.

Culturing and Test Procedures

The first step of the inventive process comprises adding protozoa and feed into test and control samples which contain inorganic buffers. The chemical substance whose toxic activity is being investigated is then added to the test samples but not to the control samples. The order of adding protozoa, feed and chemical substance is not critical. The amount of protozoa added is usually about at or below $1 \times 10^6$ per ml. If dead bacteria are used as feed, they are added at sufficient concentrations to increase the turbidity of the medium to at least 0.3 at 540 nm in a 1.0 cm pathlength tube; this corresponds to concentration of about $2.5 \times 10^9$ bacteria per ml. If live bacteria are added as feed, the inoculum level is about 0.01 to about 1% of a fully grown bacterial culture in the range of about $1 \times 10^6$ to about $1 \times 10^8$ bacteria per ml. This inoculum level does not cause any noticeable change in sample turbidity. A sample containing live bacteria is supplemented with a carbon source to support bacterial growth. The carbon sources are generally used at a final concentration of about 0.05%.

The test and control samples are then cultured at room temperature in the range from about 60°F to about 76°F for a time sufficient for the control to exhibit a significant reduction in turbidity, of about at least 50%. This reduction in turbidity generally takes about 1 to about 5 days. If the feed employs live bacteria, there is an initial increase in the turbidity. This increase is then followed by a decrease in turbidity in the control tubes as stated above. If the test chemical is not toxic to the protozoa, the turbidity decreases in the sample tube. If the test chemical is toxic to the protozoa, the turbidity will remain essentially unchanged. The size and volume of the resulting mixture are not critical, provided there is sufficient volume to permit either visual or spectrophotometrical observations of the change in turbidity of the microbial cultures. Typically, about 3 ml to about 5 ml samples were used.

Establishment of an Optimal Growth and Test Media

The concentrations of both the mineral salts and the carbon source used in the growth and test media were tested in order to determine the minimum concentrations of these materials capable of supporting bacterial and protozoan growth, while simultaneously allowing a significant distinction between toxic and non-toxic test compounds. The minimum concentrations of mineral salts and carbon source were required in order to limit the growth of the bacteria and to prevent continuous growth of the protozoa which is common when carbon and energy sources are not limited. Mineral salts typically consisted of the Bushnell-Haas broth and the carbon source was yeast extract (available from Difco Co.). A typical inorganic salts buffer employed is the Bushnell-Haas broth, which contains the following chemicals in grams per liter: about 0.05 g/l of magnesium sulfate, about 0.002 g/l of calcium chloride, about 0.01 g/l of mono-potassium phosphate, about 0.01 g/l of di-potassium phosphate, about 0.1 g/l of ammonium nitrate and about 0.005 g/l of ferric chloride.

A composition of about 0.1X Bushnell-Haas broth and about 0.05% yeast extract provided for the preferable amount of growth to distinguish between the presence and absence of actively feeding protozoa. At higher carbon concentrations, the protozoan effect on the bacterial population was insignificant because either: (1) there was sufficient carbon nutrients for continued bacterial growth, despite consumption of some of the bacteria by the protozoa; or (2) the high concentration of carbon nutrient was toxic to the protozoa and inhibited their feeding behavior. At lower carbon concentrations, insufficient bacteria growth was detected since there was insufficient nutrient concentration to support growth.

Growth Of Protozoa On Live Bacteria As A Feed

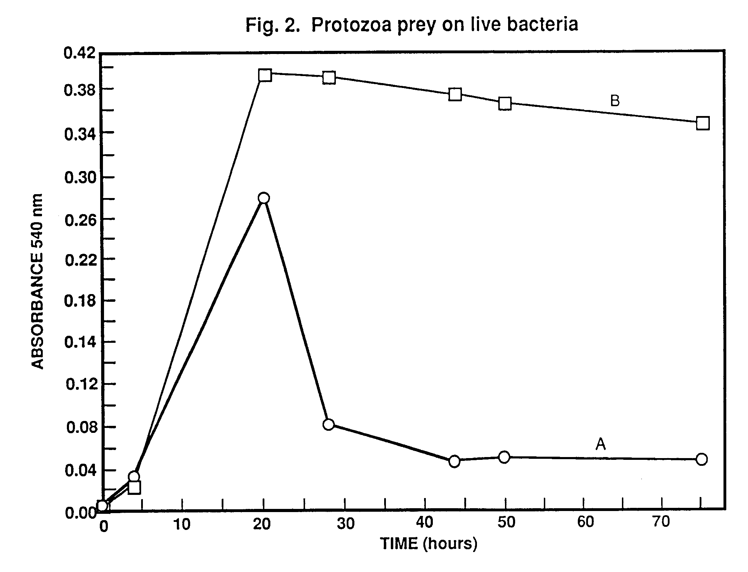

Protozoa, Anthophysa vegetans at about $1 \times 10^5$ per ml and a mixed bacteria population of soil bacteria containing but not limited to Alcaligenis and Psuedomonas at about $1 \times 10^6$ per ml were inoculated into a growth medium as described above in "Culturing and Test Procedures". The bacteria multiplied in numbers and caused an initial increase in the turbidity (absorbance) of the growth medium, generally from about 0.03 to about 0.4, as measured at 540 nm in a 1.0 cm pathlength tube. After about one day of incubation, the bacterial growth ceased due to the limitation of carbon source. Protozoa, feeding on the growing bacteria, also increased in numbers, but at a slower rate. The protozoa preyed on the bacteria, leading to a reduction in bacterial cell concentration, indicated by a reduction of turbidity. This growth sequence is illustrated in Line A of Figure 2. If the bacterial cultures were grown without added protozoa, a similar increase in turbidity took place. However, there was no significant reduction in turbidity after the growth phase was completed. This is illustrated in Line B of Figure 2.

Line A demonstrates that protozoa and live bacteria were incubated in a growth medium and the bacterial growth led to initial increase in turbidity, followed by protozoa preying on bacteria, leading to decreased turbidity of the growth medium. Line B demonstrates only bacteria incubated in growth medium. The turbidity increased due to bacterial growth and remained high for the duration of the experiment since no protozoa were present to prey on the bacteria.

Growth Of Protozoa On Dead Bacteria As A Feed

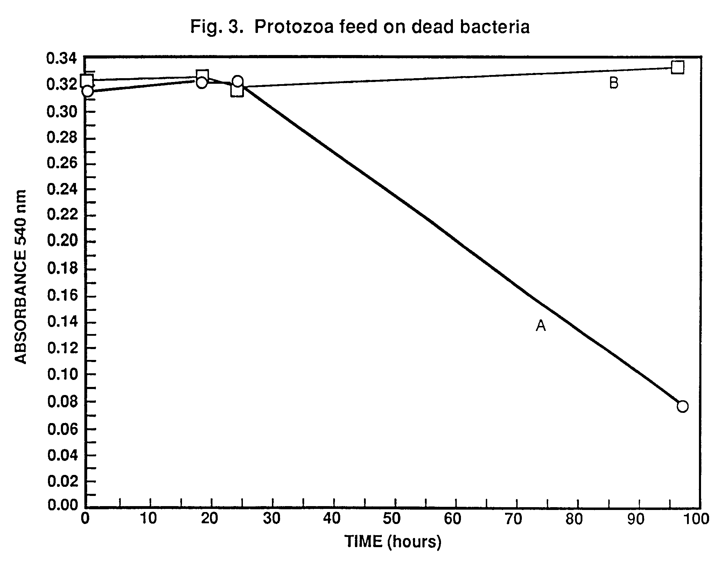

Mixed cultures of bacteria containing but not limited to Psuedomonas and Alcaligenis were taken from northern Ohio soil samples and were grown in growth media as described under "Culturing and Test Procedures". After observing maximal growth, based on the turbidity of the culture, the bacteria were killed by autoclaving at about 250ºF for about 20 minutes. The autoclaved bacteria-growth media mixture was centrifuged three times at about 13,000g for about 10 minutes. Each centrifugation cycle included a wash in growth medium devoid of the organic carbon. The autoclaved, washed bacteria was resuspended in a medium without organic carbon. Fresh protozoa inoculum of Anthophysa vegetans at about $10^5$ per ml was added to one set of test sample tubes and a control tube without added protozoa was also prepared. As shown in Line A of Figure 3, there was a decrease in initial turbidity due to consumption of the heat-killed bacterial cells. Line B of Figure 3 shows that initial turbidity of the control, without protozoa did not decrease significantly. Line A demonstrates protozoa consumed the dead bacteria resulting in a decrease in initial absorbance. Line B demonstrates that the bacterial concentration remained constant since there were no protozoa present to consume the dead bacteria.

Example 1 - Effect of test chemical on protozoa and bacterial growth

6

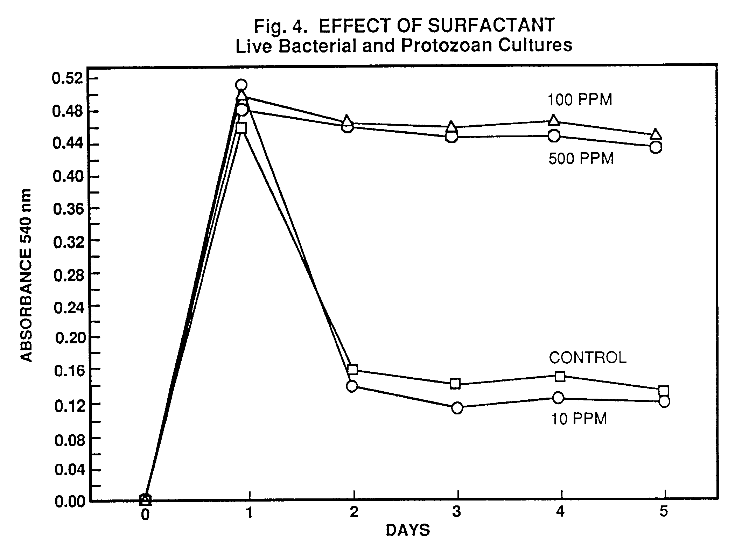

Anthophysa vegetans protozoa and live mixed population of soil bacteria containing but not limited to Psuedomonas and Alcaligenis were inoculated into a growth medium as described under "Culturing and Test Procedures". The test chemical, a phenol ethoxylate surfactant, Igepal CO 850 (GAF Chemicals Corp.), was added at different concentrations. A control without added surfactant was maintained. As shown in Figure 4, in all cases, the bacterial concentration, as measured by its absorbance at 540 nm, increased to a maximum after one day incubation at room temperature. However, at about 0 and about 10 ppm surfactant, the absorbance decreased on further incubation. When the Igepal CO 850 concentration was greater than about 100 ppm, the protozoa feeding behavior was inhibited and the protozoa did not consume the bacteria and the turbidity of the culture did not decrease significantly. The tolerance level of the protozoa, (i.e., the concentration level at which feeding inhibition is first observed) is about 100 ppm Igepal CO 850. At about 0 and about 10 ppm Igepal had no apparent effect on the protozoan feeding behavior. The protozoa preyed on the bacteria, resulting in reduced absorbance at 540 nm after two days incubation. At about 100 and about 500 ppm Igepal, the protozoan feeding behavior was inhibited. The protozoa did not prey on the bacteria, therefore, the absorbance level remained essentially constant following the initial increase.

Example 2 - Determination of effectiveness of industrial wastewater treatments

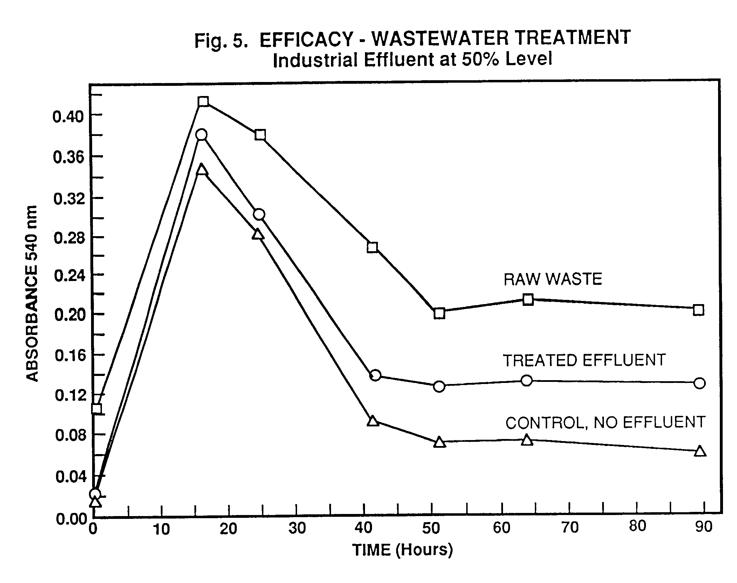

Anthophysa vegetans protozoa and live mixed population of soil bacteria containing for example Psuedomonas and Alcaligenis were inoculated into a growth medium as described under "Culturing and Test Procedures". In order to determine the efficacy of industrial wastewater treatments, samples of system effluents, which may contain many chemical compounds, were obtained and tested within 24 hours from collection. The samples were 24 hour composites of: (a) plant effluents treated by standard aerobic biodegradation, obtained at the discharge point; and (b) untreated raw wastes, as they entered the wastewater treatment system. Samples of (a) and (b) were added at about 4%, about 20%, about 50% and about 100% concentrations and a control without added effluent was maintained by adding appropriate volumes of distilled water. As shown in Figure 5, the bioassay was useful in determining the effectiveness of a biological wastewater treatment system in reducing toxicity of industrial effluents. The final turbidity was significantly higher in the raw, untreated effluent sample than in the control. This indicates that the toxicity of the effluent was sufficient to inhibit protozoan feeding behavior, allowing a substantial percentage of the bacteria to survive. Although the turbidity of the treated sample was less than that of the raw sewage sample, it was still greater than the control. This means some residual toxicity still remained after wastewater treatment.

Example 3 - Correlation between turbidity and number of live protozoa

Anthophysa vegetans protozoa and live mixed population of soil bacteria containing for example Psuedomonas and Alcaligenis were inoculated into a growth medium as described under "Culturing and Test Procedures". A phenol ethoxylate surfactant, Igepal CO 850, was added at concentrations in the range of about 0 to about 200 ppm. Turbidity measurements were taken periodically for two days. Live enumeration of the protozoa was measured after about 30 hours incubation by serial dilution of the cultures and incubation in test tubes containing the growth medium without added toxicant. Three samples were measured for each chemical concentration and the most probable number (MPN) of live protozoa was calculated. The data are shown in Table 1. Low turbidity (less than about 0.1) with high MPN's indicates the presence live protozoa which were not adversely affected by the Igepal since they consumed the turbidity-causing bacteria. Higher turbidities (greater than about 0.3) with low MPN of protozoa shows that the Igepal adversely affected the protozoa and inhibited the protozoa preying on the bacteria, so that the turbidity of the sample was not reduced significantly. This demonstrates that the number of protozoa in the test medium, in the range of about 0 to about 1,000,000 per ml, does not have a significant effect on the turbidity of the medium. Thus, only the number of bacteria determines the light absorbance on the test medium.

Example 4 - Comparison of sensitivities between present test and literature values

Anthophysa vegetans protozoa and live mixed population of soil bacteria containing for example Psuedomonas and Alcaligenis were inoculated into a growth medium as described under "Culturing and Test Procedures". Different chemicals, added at varying concentrations, were tested to determine their effect on the feeding behavior of the protozoa and their biotoxicity. The data, expressed as ppm of the lethal concentration to kill about 50% of the sample population ($LC_{50}$) are shown in Table 2. Table 2 also compares the $LC_{50}$ determined by the present invention to values reported in the scientified literature for ecotoxicity tests for these chemicals, where Daphnids rather than protozoa were used as the test species. The results demonstrate close resemblance in sensitivity to toxicants between the present invention and the Daphnid ecotoxicity tests.

Example 5 - Initial inoculation size as a factor determining protozoan sensitivity to toxicants

Anthophysa vegetans protozoa and live mixed population of soil bacteria containing for example Psuedomonas and Alcaligenis were inoculated into a growth medium as described under "Culturing and Test Procedures". Additionally, a protozoan inoculum was sequentially diluted by a factor of ten to obtain different levels of diluted inocula. A toxicant, 4-nitrophenol, was added at different concentrations to each inoculation level. The results are shown in Table 3. As the initial protozoa concentration was decreased, its sensitivity (expressed as ppm of $LC_{50}$) to the toxicant increased (i.e., its $LC_{50}$ decreased). This demonstrates that the sensitivity of the present inventive method can be altered by the initial inoculation level of the test protozoa. This increases the flexibility of the present method by allowing tailoring of the method's sensitivity according to specific requirements, such as a comparison of sensitivity to a given test organism such as different Daphnids.

Table 1

| Correlation Between Live Protozoa Concentration and Turbidity of Growth Medium | | | | | | |
|---|---|---|---|---|---|---|
| Igepal (ppm) | Turbidity[a] After Incubation (hours) | | | | | Number Live Protozoa per ml (MPN) |
| | 0 | 16 | 24 | 40 | 48 | |
| 0 | .010 | .163 | .124 | .076 | .072 | 930,000 |
| 20 | .010 | .187 | .175 | .080 | .078 | 930,000 |
| 40 | .010 | .240 | .269 | .137 | .090 | 2,000 |
| 60 | .010 | .258 | .314 | .288 | .293 | 150 |
| 75 | .005 | | .426 | .394 | .388 | 0 |
| 200 | .005 | | .383 | .363 | .357 | 0 |

(a) Measured at 540 nm in a 1.0 cm pathlength tube

## Table 2

LC$_{50}$ of Protozoa for Various Chemicals Compared to Daphnid

Data in the Literature

| Compound | LC$_{50}$ | Literature LC$^{50}$ | Ref.[a] | Organism[b] |
|---|---|---|---|---|
| | (ppm) | (ppm) | | |
| Cadmium chloride | <0.9 | 1.9–3.0 | 5 | A |
| | | 0.02–0.08 | 1 | A |
| Pentachlorophenol | <2 | 0.8–1.3 | 8 | A |
| | | 0.9–1.4 | 8 | D |
| | | 0.8–1.2 | 8 | E |
| | | 0.004 | 7 | E |
| Potassium dichromate | 2.1 | 0.003–0.13 | 3 | B |
| | | 0.35–0.93 | 5 | A |
| | | 0.1 | 8 | A |
| | | 0.2 | 8 | E |
| 4-Nitrophenol | 4.2 | 8.0 | 5 | A |
| Ethylene diamine | 7.0 | 14.0 | 5 | A |
| 2-Phenylphenol | 6.0 | 2.1 | 4 | A |
| Quinoline | 19.0 | 76.0 | 5 | A |
| p-Cresol | 21.0 | 14.0 | 4 | A |
| | | 4.9 | 5 | A |
| Aniline | 62.0 | 0.9 | 4 | A |
| | | 0.9 | 5 | A |
| Nitrobenzene | 78.0 | 60.0 | 5 | A |
| Methanol | 165.0 | nd | | |
| Toluene | 165.0 | 84.0 | 5 | A |
| Phenol | >300.0 | 21.0 | 4 | A |
| Chloroform | >300.0 | 79 | 5 | A |
| | | 66 | 2 | A |
| Ethanol | >300.0 | 12,340 | 6 | A |
| | | 6,492 | 6 | C |

(a) Reference:

    1. Hall, S.W., R.L. Paulson, L.W. Hall, Jr., and D.T. Burton, 1986, Acute Toxicity Of Cadmium And Sodium Pentachlorophenate To Daphnids And Fish. Bull. Environ. Toxicol. 37: 308-316.

    2. Cowgill, U.M., 1987, Critical Analysis Of Factors Affecting The Sensitivity Of Zooplankton And The Reproducibility Of Toxicity Test Results, Wat. Res. 21: 1453-1462.

    3. Dorn, P.B., J.H. Rodgers, Jr., K.M. Jop, J.C. Raia, and K.L. Dickson, 1987, Hexavalent Chromium As A Reference Toxicant In Effluent Toxicity Tests, Environ. Toxicol. Chem. 6: 435-444.

4. Kuhn, R., M. Pattard, K-D. Pernak, and A. Winter, 1989, Results Of The Harmful Effects Of Selected Water Pollutants (Anilines, Phenols, Aliphatic Compounds) To _Daphnia magna_, Wat. Res. 23: 495-499.

5. Kuhn, R., M. Pattard, K-D. Pernak, and A. Winter, 1989, Results Of The Harmful Effects Of Water Pollutants To _Daphnia magna_, Wat. Res. 23: 501-510.

6. Takahashi, I.T., U.M. Cowgill, and P.G. Murphy, 1987, Comparison Of Ethanol Toxicity To _Daphnia magna_ And _Ceriodaphnia dubia_ Tested At Two Different Temperatures: Static Acute Toxicity Test Results, Bull. Environ. Contam. Toxicol. 39: 229-236.

7. Hedtke, S.F., C.W. West, K.N. Allen, T.J. Norberg-King, and D.I. Mount, 1986, Toxicity Of Pentachlorophenol To Aquatic Organisms Under Naturally Varying And Controlled Environmental Conditions, Environ. Toxicol. Chem. 5: 531-542.

8. Elnabarawy, M, A.N. Welter, and R.R. Robidean, 1986, Relative Sensitivity Of Three Daphnid Species to Select Organic And Inorganic Chemicals. Environ. Toxical. Chem. 5: 393-398.

(b) Organisms compared in literature:

   A.  _Daphnia magna_

   B.  _Ceriodaphnia_ (unspecified species)

   C.  _Ceriodaphnia dubia_

   D.  _Daphnia pulex_

   E.  _Ceriodaphnia reticula_

Table 3

| Dependence of $LC_{50}$ on Protozoa Inoculum Size | |
|---|---|
| Protozoa Inocula Level | ppm $LC_{50}$ |
| 1.0X | 36 |
| 0.1X | 19.5 |
| 0.01X | 12.5 |
| 0.001X | 5.6 |
| 0.0001X | 0.46 |

**Claims**

1.  A process for determining the biotoxic effect of a chemical on organisms comprising:

    a. obtaining protozoa at concentrations insufficient to scatter greater than about 2% of incident light at about 540 nm;

    b. adding an organic feed to the protozoa to form a growing protozoa culture, wherein the feed comprises;

i) at least one substance at a concentration sufficient to scatter light of wavelengths greater than 400 nm, or

ii) at least one substance at an initial concentration insufficient to scatter light of wavelengths greater than 400 nm, but which can increase in concentration sufficiently to scatter light of wavelengths greater than 400 nm;

c. dividing the protozoa and the feed culture into a test sample and a control sample;

d. adding to the test sample a chemical agent having unknown toxic effect at a concentration sufficient to alter the feeding behavior of the protozoa;

e. culturing the test sample containing the added chemical and the control sample without the added chemical under similar conditions until more than about 70% of the light-scattering feed has been consumed in the control sample;

f. determining the concentrations of the light-scattering feed remaining in the test and control samples, wherein the determination can be carried out by either a visual examination or by measurement of absorbance at a selected wavelength; and

g. relating the relative concentrations of the remaining feed to the toxicity of the chemical agent.

2. The process of claim 1 wherein the toxicity of the chemical agent is measured using a spectrophotometer in the visible light range of 350 nm to 650 nm and the turbidity is measured at a wavelength of 540 nm by the degree of turbidity in the liquid medium containing the protozoa and the feed and when the turbidity is in the range from clearness to reduced turbidity indicates that the feeding behavior of the protozoa was unaffected or minimally affected by the added chemical and the chemical is not toxic and when the turbidity is in the range from partial to substantial turbidity in the visible range indicates the feeding behavior of the protozoa was inhibited and the chemical is toxic.

3. The process of claim 1 wherein the protozoa are selected from the group consisting essentially of Anthophysa vegetans, Labyrinthula macrocystis, Paramecium aurelia, Tetrahymena puriformis and combinations thereof and wherein the feed are selected from the group consisting of non-living organic particles, living organic particles and combinations thereof.

4. The process of claim 3 wherein the protozoa are Anthophysa vegetans.

5. The process of claim 1 wherein the protozoa can be used as a pure culture or a mixed protozoa culture and wherein the protozoa are inoculated at a concentration about at or below $1 \times 10^6$ protozoa per ml.

6. The process of claim 1 wherein the bacterial feed is live, dead or combinations thereof and wherein the non-living organic particles are selected from the group consisting of dead bacteria, cellulose granules, starch granules, oil droplets and combinations thereof and wherein the living organic particles are bacteria.

7. The process of claim 6 wherein the bacteria are selected from the group consisting of Acinetobacter, Corynebacterium, Enterobacter, Psuedomonas and combinations thereof.

8. The process of claim 3 wherein the organic particle feed is added to the sample at a concentration sufficient to increase the turbidity of the medium to at least about 0.3 at 540 nm in about a 1.0 cm pathlength tube or wherein the dead bacteria is added as feed concentrations at greater than about 2.5 $\times 10^9$ dead bacteria per ml indicated by turbidity of at least about 0.3 at 540 nm in a 1.0 cm pathlength tube or wherein live bacteria is added as feed concentrations in the range of about $1 \times 10^6$ bacteria per ml to about $1 \times 10^8$ per ml or wherein the live bacteria is added as feed concentrations less than about $7 \times 10^7$ live bacteria per ml, indicated by a turbidity of less than 0.1 at 540 nm in a 1.0 cm pathlength tube.

9. The process of claim 1 wherein the chemical agents toxicity are determined and are selected from the group consisting of pharmaceuticals, agrochemical products, chemicals, pesticides, industrial effluents, pollutants, waste, wastewater and combinations thereof.

10. The process of claim 9 wherein the chemical agents which have specific bactericidal action need to employ non-living feed in the test sample and wherein the chemical agent which has specific bactericidal action is antibiotic.

Fig. 1. Absorbance as a function of number of bacteria

EP 0 511 759 A1

Fig. 2. Protozoa prey on live bacteria

EP 0 511 759 A1

Fig. 3. Protozoa feed on dead bacteria

Fig. 4. EFFECT OF SURFACTANT
Live Bacterial and Protozoan Cultures

Fig. 5. EFFICACY - WASTEWATER TREATMENT
Industrial Effluent at 50% Level

EP 0 511 759 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | WATER RESEARCH<br>vol. 14, 1980, GB<br>pages 231 - 241;<br>G.BRINGMANN ET AL: 'Comparison of the toxicity thresholds of water pollutants to bacteria, algae, and protozoa in the cell multiplication inhibition test.'<br>* the whole document * | 1,2,6,7,9 | C12Q1/18<br>C12Q1/02 |
| A,D | Z. WASSER ABWASSER FORSCH.<br>no. 1, 1980,<br>pages 26 - 31;<br>VON GOTTFRIED BRINGMANN ET AL.: 'Bestimmung der biologischen Schadwirkung wassergefaerdender Stoffe gegen Protozoen II. Bakterienfressende Ciliaten.'<br>* the whole document * | 1,2,6,9 | |
| A,D | US-A-4 604 351 (L.AMARAL)<br>* column 1 - column 2 * | 1,2 | |
| | | | TECHNICAL FIELDS SEARCHED (Int. Cl.5)<br><br>C12Q |

The present search report has been drawn up for all claims

| Place of search | Date of completion of the search | Examiner |
|---|---|---|
| THE HAGUE | 15 JULY 1992 | HITCHEN C.E. |